Europäisches Patentamt

European Patent Office

Office européen des brevets

(19)

(11) **EP 0 804 206 B1**

(12) **FASCICULE DE BREVET EUROPEEN**

(45) Date de publication et mention
de la délivrance du brevet:
**19.09.2001   Bulletin 2001/38**

(51) Int Cl.[7]: **A61K 31/60**
// (A61K31/60, 31:16)

(21) Numéro de dépôt: **96901005.7**

(22) Date de dépôt: **02.01.1996**

(86) Numéro de dépôt international:
**PCT/FR96/00001**

(87) Numéro de publication internationale:
**WO 96/20714 (11.07.1996 Gazette 1996/31)**

(54) **COMPOSITION ANTIFONGIQUE CONTENANT DU CROTAMITON ET DE D'ALDEHYDE SALICYLIQUE**

ANTIMYKOTISCHE ZUSAMMENSETZUNG WELCHE CROTAMITON UND SALICYLALDEHYD ENTHÄLT

ANTIFUNGAL AGENT CONTAINING CROTAMITON, AND SALICYCLIC ALDEHYDE

(84) Etats contractants désignés:
**AT BE CH DE DK ES FR GB GR IE IT LI LU MC NL PT SE**

(30) Priorité:  **03.01.1995  FR 9500028**

(43) Date de publication de la demande:
**05.11.1997   Bulletin 1997/45**

(73) Titulaire: **Pierre Fabre Dermo-Cosmetique**
**92100 Boulogne (FR)**

(72) Inventeurs:
 • **FABRE, Pierre**
   **F-81106 Castres (FR)**
 • **JEANJEAN, Michel**
   **F-31320 Castanet-Tolosan (FR)**
 • **LAGARDE, Isabelle**
   **F-31520 Ramonville-Saint-Agne (FR)**
 • **COUSSE, Henri**
   **F-31860 Pins-Justaret (FR)**

 • **LAURENS, Marielle**
   **F-31750 Escalquens (FR)**

(74) Mandataire: **Ahner, Francis**
   **Cabinet Régimbeau**
   **20, rue de Chazelles**
   **75847 Paris cedex 17 (FR)**

(56) Documents cités:
   **EP-A- 0 070 525**

 • **POSTGRADATE MEDICINE, vol. v22, Juin 1979, pages 52-56,-59-62, XP002003821 ORKIN, M._MAIBACH, H. I.: "SCABIES, A CURRENT PANDEMIC"**
 • **DATABASE WPI Section Ch, Week 9340 Derwent Publications Ltd., London, GB; Class A96, AN 93-317389 XP002003822 & JP,A,05 229 949 ( ZERIA SHINYAKU KOGYO KK) , 7 Septembre 1993**

## Description

La présente invention a pour objet de nouveaux produits ayant une activité antifongique, et leurs applications, en particulier dans le traitement de dermatophyties connues sous le nom de "pied d'athlète".

Les dermatophyties sont parmi les plus répandues des maladies infectieuses de l'homme. Bien que la fréquence dans la population générale soit difficile à évaluer, les enquêtes montrent des pics de prévalences de 30 à 70 pour cent-dans des groupes humains utilisant des installations sanitaires collectives (sportifs, nageurs, collectivités scolaires ou industrielles ; mines).

L'étiologie de dermatophyties est due aux trois genres de champignon : Trichophyton, Microsporum et Epidermophyton.

Le mode de contamination est direct par l'intermédiaire du sol ou d'objets souillés qui sont les réservoirs de champignons kératinophiles, de dermatophytes anthrophiles ou zoophiles provenant des desquamations. Les dermatophytes zoophiles sont responsables de petites épidémies ou de cas isolés dans l'entourage d'un animal porteur (éleveurs, vétérinaires, propriétaires d'animaux domestiques). Certaines espèces de dermatophytes sont associées à une grande diversité d'hôtes et représentent dans beaucoup de pays, les deuxièmes ou troisièmes causes de dermatophyties humaines. Ces souches ou variétés sont probablement en train d'évoluer vers des espèces séparées, spécifiques d'un hôte particulier.

Ainsi, Trichrophyton mentagrophytes, variété interdigitale, semble devenir strictement antropophile et adapté à une niche écologique particulière dans les espaces interdigito-plantaires de l'homme où il peut vivre en saprophyte, en l'absence de lésions cliniques. D'autres espèces kératinophiles telluriques (ex : Trichophyton terrestre), suivent aussi un processus d'adaptation parasitaire chez l'animal.

La flore dermatophytique est donc flexible et se traduit par l'évolution de la répartition géographique et de la prévalence des espèces responsables des dermatophyties humaines. Les changements se produisent en l'espace de quelques décennies et sont le fait de migrations de populations, du développement des voyages, et des modifications du mode de vie. Les espèces rencontrées actuellement en France, sont différentes de celles que l'on isolait au début du siècle. Ainsi, les quatre espèces de dermatophytes les plus isolées sont : T. rubrum, T. mentagrophytes, M. canis et E. floccosum.

T. rubrum est le plus préoccupant en raison des tableaux polymorphes récidivants et rebelles qu'il occasionne. Cette espèce est connue en Europe Occidentale et en Amérique depuis la deuxième guerre mondiale ; elle y a été introduite à partir de foyers d'Asie et d'Afrique de l'Ouest.

L'interaction des dermatophytes et des bactéries résidentes, fait l'objet d'un spectre clinique particulier appelé "Athlète Foot" ou "Pied d'Athlète", qui débute par l'invasion de la couche cornée par des dermatophytes. Ce stade se manisfeste par une desquamation faible à modérée, souvent sans symptôme. Lorsque le pied parasité se trouve envahi, soit par la sueur ou l'humidité, soit en présence de chaussures serrées, ou par trop d'exercice, l'excédent d'humidité provoque une croissance exponentielle bactérienne.

L'hydratation et la grande population de germes résidents, ne sont pas suffisants pour produire la maladie par elle-même. Lorsque les espaces interdigitaux sont hyperhydratés, très peu de modifications se produisent malgré une grande croissance microbienne.

L'aggravation a lieu lorsque la couche cornée a été lésée. Au fur et à mesure que la clinique présente un tableau plus symptomatique avec augmentation de l'humidité locale, le champignon devient de plus en plus difficile à isoler, car il se situe alors dans les couches profondes du stratum corneum (qui peut atteindre des épaisseurs de 400 à 500 µm). Si l'espace interdigital s'assèche, la dermatophytose simple apparaît et le champignon redevient plus facile à isoler à partir des squames interdigitales. Ainsi, c'est cette stabilité et chronicité particulières, qui constituent le "Pied d'Athlète".

Le rôle des bactéries dans le pied d'athlète symptomatique a été déterminé à partir d'expériences dans lesquelles les patients ont été traités, soit à l'aide d'antifongiques topiques, soit d'antibiotiques topiques, ou bien avec le couple antifongique/antibiotique.

Dans le cas de dermatophytoses complexes, un antifongique tel que le "tolnaftate", apporte une amélioration modeste, car la composante principale, est microbienne. D'un autre côté, les agents fongistatiques améliorent les signes cliniques de la variété squameuse et sèche, dans laquelle le champignon est la source principale de la pathologie.

Les traitements qui, à la fois, suppriment les bactéries et les champignons, et assèchent localement les espaces interdigitaux, ont de grandes probabilités d'être les plus efficaces, car ils s'attaquent à tous les aspects du désordre.

Les malades atteints du Sida, constituent, à l'heure actuelle, une nouvelle donnée en cours d'exploitation statistique, car il semblerait que les isolements de ces germes passent de 17% avant 1986, à plus de 30% dans les trois premiers trimestres de 1987.

Une autre conséquence de ces dermatophytoses, sont les manifestations allergiques ou dermatophyties.

De très nombreuses lésions dermatologiques ont été décrites comme "éruptions secondes", chez des malades atteints de trichophyties et plus particulièrement de lésions. Leur caractère commun est d'être exemptées de tout parasite ; nous citerons le lichen trichophytique, l'érythème noueux, l'exanthème scarlatiniforme, l'érythème polymorphe, l'impétigo trichophytique. Ce sont des parakératoses, dites "infectieuses", des eczématides d'étiologie mal définie, qui requièrent l'exploitation de l'allergie fongique.

Devant l'importance grandissante de ce type de pathologies, il existe un besoin accru de molécules possédant un pouvoir antifongique et antimicrobien. D'une manière inattendue, les Demandeurs ont trouvé que le crotamiton et l'aldéhyde salicylique, présentent sur les dermatophytes, une synergie d'activité particulièrement intéressante.

C'est pourquoi la présente invention a pour objet un produit antifongique, caractérisé en ce qu'il consiste en une association synergique de crotamiton et d'aldéhyde salicylique.

Le crotamiton, ou N-éthyl-O crotonotoluidide est connu pour ses propriétés scabicides et antiprurigineuses. Il a également été proposé comme adjuvant de pénétration dans des compositions à usage topique.

L'aldéhyde salicylique ou salicylaldéhyde est un composé liquide, incolore, utilisé en parfumerie.

La Demanderesse a maintenant trouvé que ces produits ont des activités antimicrobiennes sur les bactéries, les levures et les fongi.

L'aldéhyde salicylique possède des activités antimicrobiennes sur les gram + et - de l'ordre de 0,03 - 0,06% et sur les levures inférieures à 0,01%.

Le crotamiton possède des activités antimicrobiennes du même niveau, sur les bactéries et bien meilleures encore sur les levures et fongi (bactéries 0,5% +/- 0,2% p/v, fongi et levures 0,1 +/- 0,05%).

L'association des deux molécules Aldéhyde salicylique / Crotamiton est intéressante à double titre :

- elles présentent des spectres antifongiques complémentaires sur les différentes levures impliquées dans le pied d'athlète,
- elles possèdent une synergie d'action particulièrement importante.

De plus, elle offre une complémentarité très intéressante dans leur mode d'action.

Le Crotamiton est fongicide et, à ce titre, il agit immédiatement.

L'Aldéhyde salicylique fongistatique constitue l'antifongique de choix pour une action plus prolongée.

L'association, dans des proportions définies, des deux molécules entraîne la potentialisation de l'effet inhibiteur de croissance observé pour chaque composé.

De préférence, le rapport en poids des concentrations crotamiton / aldéhyde salicylique est compris entre 60 et 0,1. Ce rapport est avantageusement compris entre 2 et 0,5 et sera adapté lors de la formulation de ces produits pour leur administration, en fonction des inhibitions possibles engendrées par le choix des excipients.

En effet l'invention a également pour objet une composition antifongique, par exemple cosmétologique et/ou pharmaceutique, caractérisée en ce qu'elle contient une association synergique antifongique de crotamiton et d'aldéhyde salicylique. Les rapports des concentrations seront comme définis précédemment, de bons résultats étant obtenus avec les produits formulés à la même concentration finale.

La synergie peut être mise-en évidence par le calcul des index FIC (Fractional Inhibitory Concentration) ou FBC (Fractional Bactericidal Concentration) des produits. Pour cela, on mesure la valeur de la CMF (ou concentration minimale fongistatique), ou de la CMφ (ou concentration minimale fongicide), des principes actifs à tester vis-à-vis d'un type de germe.

Le FIC d'un produit A est défini comme :

$$FIC_A \quad \frac{CMF \text{ du produit A en association}}{CMF \text{ du produit A seul}}$$

L'association de deux antifongiques A et B est synergique si la somme FIC des rapports ($FIC_A + FIC_B$) est inférieure ou égale à 0,75.

Plus la valeur FIC est faible, plus la synergie est importante.

On considère qu'il y a simple additivité pour des valeurs de FIC comprises entre 0,75 et 1,1, et indifférence dans l'intervalle compris entre 1,1 et 2 ; au-delà, l'association est notée antagoniste.

Après détermination des niveaux de concentrations minimales inhibitrices pour chaque souche, les associations entre les différents principes actifs sont testées.

$$Le \ FIC \ Index = \frac{CMI \text{ crotamiton associé}}{CMI \text{ crotamiton seul}} + \frac{CMI \text{ salicylaldéhyde associé}}{CMI \text{ salicylaldéhyde seul}}$$

est calculé.

La synergie des activités fongistatiques a été vérifiée notamment sur le genre Trichophyton et particulièrement, les espèces mentagrophytes, interdigitale et rubrum.

Les compositions selon l'invention peuvent être des compositions à usage topique, comprenant des excipients pharmaceutiquement acceptables.

Ces compositions pourront également être formulées pour des applications sur des supports susceptibles de constituer des réservoirs pour une réinfestation, tels que les chaussures, etc.

Elles peuvent se présenter sous forme de lotion, suspension, émulsion, crème, poudre, avec des supports pharmaceutiquement acceptables. Elles peuvent être conditionnées en aérosol ou pulvérisateur.

Les excipients de consistance, stabilisant, fluididant, conservateurs seront adaptés par l'homme du métier.

De par sa nature, la solution est la forme la plus pénétrante et offre une action curative immédiate. Sa mise en oeuvre simple fait souvent appel au badigeonnage dans les espaces interdigitaux.

Toutefois, selon la nature des solvants vecteurs utilisés la solution peut, dans certains cas, générer une douleur ou une irritation simultanée à l'application sur les tissus nécrosés par l'affection.

La forme poudre est particulièrement intéressante pour sa facilité d'utilisation et sa durée d'action.

D'une répartition directe dans la chaussure, elle permet son assainissement et assure un contact prolongé avec le pied et les espaces interdigitaux.

La poudre présente un pouvoir rémanent important offrant ainsi une action curative immédiate et une action préventive à plus long terme, en rallongeant le temps de contact des actifs par une libération prolongée.

Grâce à son absence de pouvoir pénétrant, la poudre reste en surface des tissus lésés sans induire de contact douloureux comme le font souvent les solvants traditionnels.

Le choix des excipients vecteurs doit tenir compte de leur aptitude à faire passer les molécules de salicylaldéhyde de l'état liquide à l'état pulvérulent.

Selon l'un des modes de mise en oeuvre de l'invention, l'aldéhyde salicylique est associé à des cyclodextrines, en particulier une β-cyclodextrine.

En effet, les Demandeurs ont mis en évidence l'intérêt particulier de l'utilisation de β-cyclodextrine pour encapsuler et véhiculer l'aldéhyde salicylique. Son affinité pour les molécules lipophiles la rend particulièrement adaptée à la préparation de composition antifongique sous forme de poudre.

Les cyclodextrines sont des composés cycliques naturels consistant en motifs $6\alpha$, $7\beta$ ou $8\gamma$ (1-->4) D-glucopyranosidiques.

Le recours à une β-cyclodextrine présente notamment les avantages suivants :

- elle transforme à l'état de poudre un principe actif liquide,
- elle atténue l'odeur marquée du salicylaldéhyde,
- elle libère progressivement son contenu par hydrolyse enzymatique induisant ainsi une activité prolongée du principe actif qu'elle contient.

La β-cyclodextrine utilisée pour piéger l'aldéhyde salicylique a une dimension particulaire de 50 $\mu$ et une densité proche de 0,70.

Le mélange peut être effectué selon les proportions suivantes :

| ALDEHYDE SALICYLIQUE | 10% |
|---|---|
| β-CYCLODEXTRINE | 90% |

La densité, la surface spécifique et la dimension des particules sont également des critères importants dont le choix déterminera les qualités du produit final.

La densité joue un rôle fondamental sur le comportement du mélange et notamment sur son homogénéité ou son pouvoir d'écoulement.

Une densité, la plus faible possible, permet d'augmenter le volume du mélange, facilite son homogénéisation et diminue fortement les phénomènes de tassement.

La surface spécifique définit le pouvoir couvrant de la poudre et, par conséquent, son efficacité à faible dose.

La finesse particulaire est une qualité nécessaire à une bonne circulation des poudres dans -leur système de distribution, plus particulièrement dans un aérosol.

Elle intervient également sur leur répartition, sur la surface à traiter et évite les défauts de dosage induits par une hétérogénéité du mélange.

Le crotamiton étant naturellement sous forme liquide, il est nécessaire de le mettre sous forme sèche pour la formuler sous forme de poudre. La poudre utilisée doit présenter des qualités de neutralité chimique, finesse et pouvoir absorbant.

De manière avantageuse le crotamiton est adsorbé sur des particules de silice microporeuse ; celle-ci a une lipophilie marquée.

Grâce à sa surface spécifique très élevée (plus de 300 cm$^2$ au gramme) cette silice microporeuse permet un captage particulièrement efficace du crotamiton dont elle va augmenter le contact avec les tissus et optimiser son activité fongicide immédiate.

En outre, en raison de sa très faible densité (inférieure à 0,30) et de son extrême finesse, cette silice microporeuse permet d'obtenir un mélange final plus expansé, possédant d'excellentes qualités d'écoulement et de dispersion. La granulométrie est avantageusement inférieure à 10 μm, de préférence inférieure à environ 5 μm.

Les compositions selon l'invention peuvent également contenir au moins un autre principe actif, notamment à activité antiseptique, antifongique, antibactérienne, antisudorale, tonifiante, cicatrisante etc.

Des substances convenant particulièrement sont les huiles essentielles obtenues par exemple à partir de girofle, géranium, verveine, menthe, lavande.

Un mélange de crotamiton et d'huile essentielle peut être adsorbé sur la silice microporeuse dans les proportions suivantes :

| HUILE ESSENTIELLE | 15 g |
|---|---|
| CROTAMITON | 35 g |
| SILICE MICRONISEE | 50 g |

Des compositions conformes à l'invention sont préparées avec du crotamiton à une concentration finale comprise entre environ 0,1 et 3% par rapport au poids total de la composition ; l'aldéhyde salicylique est avantageusement présent à une concentration finale comprise entre environ 0,05 et 1% par rapport au poids.

L'invention comprend une méthode pour maintenir l'hygiène et préserver l'aspect de la peau, qui consiste à appliquer un produit contenant une association synergique de crotamiton et d'aldéhyde salicylique selon l'invention.

L'invention a également pour objet à titre de médicament, un produit antifongique ou une composition telle que définie précédemment ; plus particulièrement, le médicament est destiné au traitement des infections fongiques et des dermatomycoses.

Les exemples qui suivent sont destinés à illustrer l'invention sans aucunement en limiter la portée.

**Exemple 1 :**

Les activités antifongiques du crotamiton et du salicylaldéhyde ont été recherchées sur Trichophyton mentagrophytes.

Les concentrations minimales fongistatiques des produits seuls et associés sont présentées ici.

**Matériels et Méthode**

* Souche

  - Trichophyton mentagrophytes n° 8611

* Milieux de culture

  - Bouillon Sabouraud
  - Gélose Sabouraud
  - Eau distillée stérile

* Produits

  - Crotamiton (Borhinger, lot-209313)
  - Salicylaldéhyde Prolabo Rectapur encapsulé dans β cyclodextrines = formule aldéhydique

Les solutions-mère aqueuses des produits suivants ont été testées pour leur meilleure solubilité et sans artéfact d'activité antifongique :

- Crotamiton : 0,2% dans 10% PEG 200
- Solution aqueuse à 10% aldhéhyde salicylique.

Les essais sont réalisés plusieurs fois car ces produits sont très volatils, rendant la lecture des tests très difficile.

Une détermination des concentrations minimales fongistatiques des trois produits se réalise en premier vis-à-vis d'une solution de Trichophyton mentagrophytes calibrée à $10^6$ spores par ml.

Les FIC Index des associations des produits en binaire ont été étudiés :

\* Crotamiton - aldhéhyde salicylique

$$\text{FIC Index} = \frac{\text{CMFA ass.}}{\text{CMFA seul}} + \frac{\text{CMFB ass}}{\text{CMFB seul}}$$

FIC Index < 0,75 indique une synergie d'activité.

Les tableaux ci-dessous indiquent les CMF = Concentrations Minimales Fongistatiques trouvées lors des essais.

A- Produits seuls

| produit<br><br>souche | Crotamiton | Formule Aldéhydique7 |
|---|---|---|
| T.mentagrophytes | 0,1% | 0,15% |

\* Crotamiton - Formule aldéhydique

| | Seul | Associés | FIC |
|---|---|---|---|
| Crotamiton | 0,1% | 0,003% | |
| Formule aldéhydique | 0,15% | 0,04% | 0,28 |

On observe que les produits utilisés seuls, présentent une concentration minimale inhibitrice fongistatique intéressante, sans toutefois pouvoir les comparer à des imidazolés ou des antibiotiques antifongiques ou des allylaminés réservés au domaine médical.

Ceci justifie l'intérêt de rechercher à améliorer leur performance antifongique en les associant.

Le Crotamiton et la formule encapsulée d'aldéhyde salicylique se synergisent fortement avec un Fic de 0,28.

En effet, il faut 30 fois moins de Crotamiton et 3 fois moins de la formule aldéhydique à 10% d'aldéhyde pour obtenir le même effet antifongique que les produits utilisés seuls.

Les produits doivent être formulés à même concentration finale.

Les concentrations proposées dans les formules seront en fonction des inhibitions potentielles des excipients choisis.

**Exemple 2 : solution antifongique**

| | |
|---|---|
| ALDEHYDE SALICYLIQUE | 0,05 à 1% |
| CROTAMITON | 0,1 à 3% |
| N-HYDROXYETHYL ACETAMIDE | 1% |
| HUILES ESSENTIELLES en mélange (Géranium, Girofle, Verveine) | 1% |
| ETHOXYDIGLYCOL | 10 ml |
| PEG-300 | 10 g |
| ALCOOL 95 | 50 ml |
| EAU PURIFIEE QSP | 100 ml |

**Exemple 3 : vaporisateur pompe**

| | |
|---|---|
| ALDEHYDE SALICYLIQUE | 0,05 à 1% |
| CROTAMITON | 0,1 à 3% |
| ESSENCE DE GIROFLE | 0,75% |
| N-HYDROXYETHYL ACETAMIDE | 1% |
| ETHOXYDIGLYCOL | 10 ml |
| METHYLAL (ou DIMETHOXYMETHANE) | 15 ml |
| ALCOOL ABSOLU | 35 ml |
| EAU PURIFIEE QSP | 100 ml |

La poudre est la forme la plus intéressante d'un point de vue pratique et pour sa durée d'action.

**Exemple 4**

. Préparation d'un mélange de crotamiton et d'huile essentielle :

| | |
|---|---|
| HUILE ESSENTIELLE | 15 g |
| CROTAMITON | 35 g |
| SILICE MICRONISEE | 50 g |

. Poudre traitante :

| | |
|---|---|
| CROTAMITON | 0,1 à 3% |
| ALDEHYDE SALICYLIQUE | 0,05 à 1% |
| ESSENCE DE GIROFLE | 0,3 à 1% |
| β-CYCLODEXTRINE (50 μ) | 0,5 à 10% |
| SILICE MICROPOREUSE (5 μ) | 0,5 à 5% |
| TALC        QSP | 100 g |

**Exemple 5 : Poudre traitante anti-transpirante**

| | |
|---|---|
| CROTAMITON | 0,1 à 3% |
| ALDEHYDE SALICYLIQUE | 0,05 à 1% |
| ESSENCE DE GIROFLE | 0,3 à 1% |
| β-CYCLODEXTRINE (50 μ) | 0,5 à 10% |

(suite)

| SILICE MICROPOREUSE (5 μ) | 0,5 à 5% |
|---|---|
| HYDROXYCHLORURE d'ALUMINIUM | 5 g |
| TALC      QSP | 100 g |

**Revendications**

1.  Produit antifongique, **caractérisé en ce qu'**il consiste en une association synergique de crotamiton et d'aldéhyde salicylique.

2.  Produit antifongique selon la revendication 1, **caractérisé en ce que** le rapport des concentrations- crotamiton / aldéhyde salicylique est compris entre 60 et 0,1.

3.  Produit antifongique selon l'une des revendications 1 ou 2, **caractérisé en ce que** le rapport crotamiton / aldéhyde salycylique est compris entre 2 et 0,5.

4.  Produit antifongique selon l'une des revendications 1 à 3, **caractérisé en ce que** l'aldéhyde salicylique est associé à des cyclodextrines, en particulier une β-cyclodextrine.

5.  Produit antifongique selon l'une des revendications 1 à 4, **caractérisé en ce que** le crotamiton est adsorbé sur des particules de silice microporeuse.

6.  Composition antifongique, **caractérisée en ce qu'**elle contient un produit antifongique selon l'une des revendications 1 à 5 et des excipients adaptés à l'administration sous forme topique.

7.  Composition selon la revendication 6, **caractérisée en ce qu'**elle contient des supports permettant sa formulation sous forme de poudre.

8.  Composition selon la revendication 7, **caractérisée en ce que** la poudre présente une granulométrie inférieure à 10 μm, de préférence inférieure à environ 5 μm.

9.  Composition selon l'une des revendications 6 à 8, **caractérisée en ce que** l'aldéhyde salicylique est encapsulé dans des β-cyclodextrines.

10. Composition selon l'une des revendications 6 à 9, **caractérisée en ce qu'**elle contient au moins un autre principe actif.

11. Composition selon l'une des revendications 6 à 10, **caractérisée en ce qu'**elle contient en outre au moins une huile essentielle.

12. Composition selon l'une des revendications 6 à 11, **caractérisée en ce que** le crotamiton est à une concentration finale comprise entre 0,1 et 3% par rapport au poids total de la composition.

13. Composition selon l'une des revendications 6 à 12, **caractérisée en ce que** l'aldéhyde salicylique est présent à une concentration finale comprise entre 0,05 et 1% par rapport au poids total de la composition.

14. Composition selon l'une des revendications 6 à 13, **caractérisée en ce qu'**elle contient un support pharmaceutiquement acceptable.

15. A titre de médicament, produit antifongique selon l'une des revendications 1 à 5.

16. Utilisation d'un produit selon l'une des revendications 1 à 5 pour la préparation d'un médicament destiné au traitement des infections fongiques.

**Claims**

1. Antifungal product, **characterized in that** it consists of a synergistic combination of crotamiton and salicylic aldehyde.

2. Antifungal product according to Claim 1, **characterized in that** the ratio of the crotamiton/salicylic aldehyde concentrations is between 60 and 0.1.

3. Antifungal product according to either of Claims 1 and 2, **characterized in that** the crotamiton/salicylic aldehyde ratio is between 2 and 0.5.

4. Antifungal product according to one of Claims 1 to 3, **characterized in that** the salicylic aldehyde is combined with cyclodextrins, in particular a β-cyclodextrin.

5. Antifungal product according to one of Claims 1 to 4, **characterized in that** the crotamiton is adsorbed onto particles of microporous silica.

6. Antifungal composition, **characterized in that** it contains an antifungal product according to one of Claims 1 to 5 and excipients suitable for administration in topical form.

7. Composition according to Claim 6, **characterized in that** it contains carriers allowing its formulation in powdered form.

8. Composition according to Claim 7, **characterized in that** the powder has a particle size of less than 10 μm, preferably of less than about 5 μm.

9. Composition according to one of Claims 6 to 8, **characterized in that** the salicylic aldehyde is encapsulated in β-cyclodextrins.

10. Composition according to one of Claims 6 to 9, **characterized in that** it contains at least one other active ingredient.

11. Composition according to one of Claims 6 to 10, **characterized in that** it contains, in addition, at least one essential oil.

12. Composition according to one of Claims 6 to 11, **characterized in that** the crotamiton is at a final concentration of between 0.1 and 3% relative to the total weight of the composition.

13. Composition according to one of Claims 6 to 12, **characterized in that** the salicylic aldehyde is present at a final concentration of between 0.05 and 1% relative to the total weight of the composition.

14. Composition according to one of Claims 6 to 13, **characterized in that** it contains a pharmaceutically acceptable carrier.

15. Antifungal product according to one of Claims 1 to 5, as a medicament.

16. Use of a product according to one of Claims 1 to 5 for the preparation of a medicament intended for the treatment of fungal infections.


**Patentansprüche**

1. Antifungus-Produkt, **dadurch gekennzeichnet, dass** es aus einer synergistischen Vereinigung von Crotamiton und Salicylaldehyd besteht.

2. Antifungus-Produkt nach Anspruch 1, **dadurch gekennzeichnet, dass** das Verhältnis der Konzentrationen von Crotamiton/Salicylaldehyd zwischen 60 und 0,1 eingeschlossen ist.

3. Antifungus-Produkt nach einem der Ansprüche 1 oder 2, **dadurch gekennzeichnet, dass** das Verhältnis Crotamiton/Salicylaldehyd zwischen 2 und 0,5 eingeschlossen ist.

4. Antifungus-Produkt nach einem der Ansprüche 1 bis 3, **dadurch gekennzeichnet, dass** der Salicylaldehyd mit Cyclodextrinen, insbesondere einem β-Cyclodextrin, vereinigt ist.

5. Antifungus-Produkt nach einem der Ansprüche 1 bis 4, **dadurch gekennzeichnet, dass** das Crotamiton auf Teilchen aus mikroporösem Kieselsäureanhydrid adsorbiert ist.

6. Antifungus-Zusammensetzung, **dadurch gekennzeichnet, dass** sie ein Antifungus-Produkt nach einem der Ansprüche 1 bis 5 und Hilfsstoffe enthält, die an die Verabreichung in topischer Form angepasst sind.

7. Zusammensetzung nach Anspruch 6, **dadurch gekennzeichnet, dass** sie Träger enthält, welche ihre Formulierung in Pulverform gestatten.

8. Zusammensetzung nach Anspruch 7, **dadurch gekennzeichnet, dass** das Pulver eine Teilchengröße unterhalb von 10 μm, vorzugsweise unterhalb von etwa 5 μm, aufweist.

9. Zusammensetzung nach einem der Ansprüche 6 bis 8, **dadurch gekennzeichnet, dass** der Salicylaldehyd in β-Cyclodextrinen eingekapselt ist.

10. Zusammensetzung nach einem der Ansprüche 6 bis 9, **dadurch gekennzeichnet, dass** sie mindestens einen anderen Wirkstoff enthält.

11. Zusammensetzung nach einem der Ansprüche 6 bis 10, **dadurch gekennzeichnet, dass** sie darüber hinaus mindestens ein ätherisches Öl enthält.

12. Zusammensetzung nach einem der Ansprüche 6 bis 11, **dadurch gekennzeichnet, dass** das Crotamiton in einer Endkonzentration von 0,1 bis 3%, bezogen auf das Gesamtgewicht der Zusammensetzung, vorliegt.

13. Zusammensetzung nach einem der Ansprüche 6 bis 12, **dadurch gekennzeichnet, dass** der Salicylaldehyd in einer Endkonzentration von 0,05 bis 1%, bezogen auf das Gesamtgewicht der Zusammensetzung, vorliegt.

14. Zusammensetzung nach einem der Ansprüche 6 bis 13, **dadurch gekennzeichnet, dass** sie einen pharmazeutisch annehmbaren Träger enhält.

15. Antifungus-Produkt nach einem der Ansprüche 1 bis 5 als Medikament.

16. Verwendung eines Produkts nach einem der Ansprüche 1 bis 5 für die Herstellung eines Medikaments, das zur Behandlung von Pilzinfektionen bestimmt ist.